# EUROPEAN PATENT APPLICATION

(11) **EP 4 667 475 A1**
(43) Date of publication of application: **24.12.2025**
(21) Application number: 24756709.2
(22) Date of filing: 02.02.2024
(51) Int. Cl.: C07F 9/10, A61K 31/7088, A61K 47/24, A61K 47/28, A61K 48/00

(54) **PH-RESPONSIVE PHOSPHOLIPID**

(30) Priority: 14.02.2023 JP 2023021016
(71) Applicant: Shizuoka Prefectural University Corporation, Shizuoka-shi, Shizuoka 422-8526 (JP); Nippon Fine Chemical Co., Ltd., Osaka-shi, Osaka 541-0051 (JP)
(72) Inventor: ASAI, Tomohiro, Shizuoka-shi, Shizuoka 422-8526 (JP); KOGE, Tomoyuki, Takasago-shi, Hyogo 676-0074 (JP); FUKATA, Naofumi, Takasago-shi, Hyogo 676-0074 (JP); MAEDA, Noriyuki, Takasago-shi, Hyogo 676-0074 (JP); KUROSAKI, Toshio, Takasago-shi, Hyogo 676-0074 (JP)
(74) Representative: Gilani, Anwar
(86) International application number: PCT/JP2024/003597
(87) International publication number: WO 2024/171859

(57) **Abstract**

The present invention provides a lipid particle that is not positively charged at a pH of the body fluid (typically in the neutral range), and that enables more efficient onset of the effect of a medicinal substance encapsulated in the lipid particle; and a lipid for forming the lipid particle.

The present invention provides a phospholipid represented by formula (1): 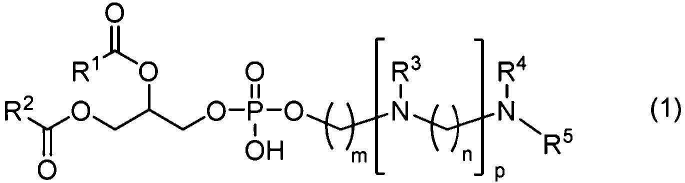 wherein R¹ and R² are the same or different, and each represents a chain hydrocarbon group; R³, R⁴, and R⁵ are the same or different, and each represents a C₁-C_{S} hydrocarbon group; m represents 1 or 2; n represents 1 or 2; and p represents a natural number of 1 to 3.

## Description

### Technical Field

The present invention relates to a pH-responsive phospholipid.

### Background Art

Recent years have seen enormous promise in messenger RNA (mRNA) as fascinating pharmaceutical seeds. An extremely sophisticated delivery system is required for externally administered mRNA to exhibit its intrinsic activity *in vivo.* This is because RNA is readily degraded by nucleases, and poorly penetrates cell membranes. Thus, the commercial viability of mRNA pharmaceutical products inevitably involves the development of a delivery system.

A known delivery system for medicinal substances, such as RNA, is administration of a medicinal substance encapsulated in a lipid particle. However, administering a negatively charged nucleic acid typically involves the use of a positively charged lipid to cause electrostatic interaction; this raises concerns regarding cytotoxicity (PTL 1).

### Citation List

### Patent Literature

PTL 1: JP2016-023147A

### Summary of Invention

### Technical Problem

The present inventors focused on the fact that a lipid particle that is not positively charged at a pH of the body fluid (typically in the neutral range) can reduce cytotoxicity.

An object of the present invention is to provide a lipid particle that is not positively charged at a pH of the body fluid (typically in the neutral range), and that enables more efficient onset of the effect of a medicinal substance encapsulated in the lipid particle; and to provide a lipid for forming the lipid particle. An object of the present invention is to further provide a lipid particle of a size that can more efficiently encapsulate a medicinal substance and/or that is suitable for more efficient delivery of a medicinal substance; and to provide a lipid for forming the lipid particle.

### Solution to Problem

The present inventors conducted extensive research to achieve the objects, and found that a phospholipid represented by formula (1) can form a lipid particle that is not positively charged at a pH of the body fluid (typically in the neutral range), and that enables more efficient onset of the effect of a medicinal substance encapsulated in the lipid particle.

Specifically, the present invention includes the following embodiments.
1. A phospholipid represented by formula (1): 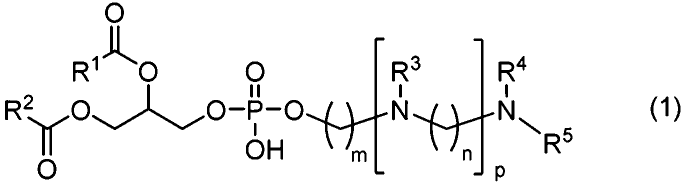 wherein R¹ and R² are the same or different, and each represents a chain hydrocarbon group; R³, R⁴, and R⁵ are the same or different, and each represents a C₁-C_{S} hydrocarbon group; m represents 1 or 2; n represents 1 or 2; and p represents a natural number of 1 to 3.
2. The phospholipid according to Item 1, wherein R³, R⁴, and R⁵ are the same or different, and each represents a C₁-C₃ hydrocarbon group.
3. The phospholipid according to Item 1 or 2, wherein m and n are 2.
4. The phospholipid according to any one of Items 1 to 3, wherein p is 1.
5. The phospholipid according to any one of Items 1 to 4 wherein R¹ and R² are the same or different, and each represents a C₁₂-C₂₄ chain hydrocarbon group.
6. The phospholipid according to any one of Items 1 to 5, wherein the chain hydrocarbon group is an unsaturated chain hydrocarbon group.
7. A phospholipid salt that is a salt of the phospholipid of any one of Items 1 to 6.
8. A lipid particle comprising the phospholipid of any one of Items 1 to 6 (phospholipid A).
9. The lipid particle according to Item 8, in which a medicinal substance is encapsulated.
10. The lipid particle according to Item 9, wherein the medicinal substance is a polynucleotide.
11. The lipid particle according to Item 10, wherein the polynucleotide is messenger RNA.
12. The lipid particle according to any one of Items 8 to 11, wherein the lipid particle comprises a sterol.
13. The lipid particle according to any one of Items 8 to 12, wherein the lipid particle is free of a polyethylene glycol (PEG) lipid.
14. The lipid particle according to any one of Items 8 to 13, wherein the lipid particle further comprises a phospholipid other than phospholipid A (phospholipid B).
15-1. An alcohol solution comprising the phospholipid of any one of Items 1 to 6.
15-2. An alcohol solution comprising the phospholipid salt of Item 7.
16. The alcohol solution according to Item 15, wherein an alcohol in the alcohol solution is ethanol.
17. A method for producing a lipid particle, the method comprising mixing the alcohol solution of Item 15 or 16 with an acidic aqueous solution containing a water-soluble medicinal substance.
18. A medical drug comprising the lipid particle of any one of Items 8 to 14.

### Advantageous Effects of Invention

The present invention provides a lipid particle that is not positively charged at a pH of the body fluid (typically in the neutral range), and that enables more efficient onset of the effect of a medicinal substance encapsulated in the lipid particle; and a lipid for forming the lipid particle. This enables a medicinal substance (e.g., a polynucleotide, such as mRNA) to more efficiently exert its effect, while reducing cytotoxicity.

### Brief Description of Drawings

Fig. 1 shows the results of a luciferase expression test, and shows the results of imaging using Examples 3 and 4.
Fig. 2 shows the results of a luciferase expression test, and is a graph of the total luminescence of Examples 3 and 4.
Fig. 3 shows the results of a luciferase expression test, and shows the results of imaging using Comparative Examples 2 and 3.
Fig. 4 shows the results of a luciferase expression test, and is a graph of the total luminescence of Comparative Examples 2 and 3.
Fig. 5 shows the particle size distribution of the lipid particle solution produced in Example 5.
Fig. 6 shows the particle size distribution of the lipid particle solution produced in Comparative Example 4.
Fig. 7 shows the evaluation results of the biodistribution of lipid particles 4 hours after administration of lipid particle solutions. Fig. 7 shows the evaluation results of the distribution in the heart, lung, liver, kidney, spleen, left thigh muscle, blood plasma, right subiliac lymph node (A), and left subiliac lymph node (B).
Fig. 8 shows the evaluation results of the biodistribution of lipid particles 24 hours after administration of lipid particle solutions. Fig. 8 shows the evaluation results of the distribution in the heart, lung, liver, kidney, spleen, left thigh muscle, blood plasma, right subiliac lymph node (A), and left subiliac lymph node (B).
Fig. 9 is a diagram in which the left thigh muscle, right subiliac lymph node (A), and left subiliac lymph node (B) are deleted from Fig. 7, and the values on the vertical axis are changed.
Fig. 10 is a diagram in which the left thigh muscle, right subiliac lymph node (A), and left subiliac lymph node (B) are deleted from Fig. 8, and the values on the vertical axis are changed.

### Description of Embodiments

In the present specification, the terms "comprise," "contain," and "include" include the concepts of "comprise," "contain," "include" "consist essentially of," and "consist of." In the present specification, upper and lower limits of numerical ranges indicated with numerals before and after "to" mean that the lower limit is X or more and the upper limit is Y or less. For example, the phrase "α to β" means that the numerical range is α or more and β or less.

### 1. Phospholipid

The present invention, according to one embodiment thereof, relates to a phospholipid represented by formula (1): 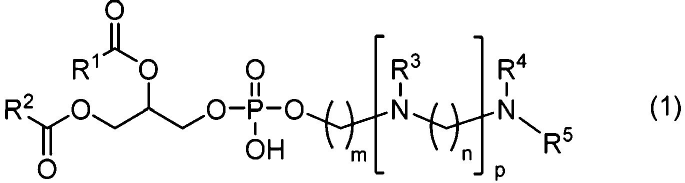 wherein R¹ and R² are the same or different, and each represents a chain hydrocarbon group; R³, R⁴, and R⁵ are the same or different, and each represents a C₁-C_{S} hydrocarbon group; m represents 1 or 2; n represents 1 or 2; and p represents a natural number of 1 to 3 (which may be referred to as "the phospholipid of the present invention" in the present specification). The following describes this phospholipid.

In formula (1), R¹ or R² is not particularly limited as long as they are the same or different, and each represents a chain hydrocarbon group; and the chain hydrocarbon group includes both linear and branched chain hydrocarbon groups. The number of carbon atoms of the chain hydrocarbon group is not particularly limited, as long as it is a number that allows the formation of lipid particles. For example, the number of carbon atoms is 4 to 30, preferably 8 to 26, more preferably 12 to 24, even more preferably 14 to 20, and still even more preferably 15 to 19. The chain hydrocarbon group includes both saturated chain hydrocarbon groups and unsaturated hydrocarbon groups. The chain hydrocarbon group is preferably an unsaturated chain hydrocarbon group, more preferably an unsaturated chain hydrocarbon group containing a double bond, and even more preferably an unsaturated chain hydrocarbon group containing only one double bond. Examples of chain hydrocarbon groups include butyl, pentyl, hexyl, heptyl, octyl, nonyl, decyl, undecyl, tridecyl, tetradecyl, pentadecyl, 9-pentadecenyl, hexadecyl, heptadecyl, cis-9-heptadecenyl, 11-heptadecenyl, cis,cis-9,12-heptadecadienyl, 9,12,15-heptadecanetrienyl, 6,9,12-heptadecanetrienyl, 9,11,13-heptadecanetrienyl, nonadecyl, 8,11-nonadecadienyl, 5,8,11-nonadecatrienyl, 5,8,11,14-nonadecatetraenyl, heneicosyl, tricosyl, cis-15-tricosenyl, pentacosyl, heptacosyl, nonacosyl, and the like.

In formula (1), it is preferable that at least one of R¹ and R² is an unsaturated chain hydrocarbon group, and it is more preferable that both of them are unsaturated chain hydrocarbon groups.

In formula (1), R³, R⁴, and R⁵ are the same or different, and each represents a C₁-C_{S} hydrocarbon group. The hydrocarbon group is not particularly limited, as long as it is a monovalent hydrocarbon group and has 1 to 5 carbon atoms. The hydrocarbon group is preferably a chain hydrocarbon group, and more preferably an alkyl group. The number of carbon atoms of the hydrocarbon group is preferably 1 to 3, more preferably 1 to 2, and even more preferably 1. Examples of hydrocarbon groups include methyl, ethyl, propyl, butyl, pentyl, and the like, with methyl being preferable.

In formula (1), it is preferable that at least one of R³, R⁴, and R⁵ is methyl, and it is more preferable that all of them are methyl.

m is preferably 2.

n is preferably 2.

m and n are both preferably 2.

p is preferably 1 or 2. From the standpoint of cytotoxicity, p is more preferably 1.

The phospholipid of the present invention can be synthesized by various methods. The compound of the present invention can be synthesized, for example, in accordance with or with reference to the following reaction scheme: 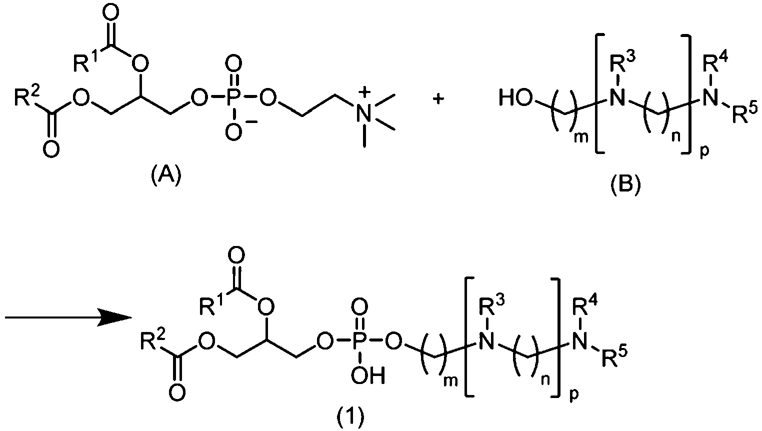 wherein R¹, R², R³, R⁴, R⁵, m, n, and p are as defined above.

In this reaction, the compound represented by formula (A) is reacted with the compound represented by formula (B) in the presence of phospholipase D, thereby preparing the compound represented by formula (1).

From the standpoint of yield etc., the amount of the compound represented by formula (B) for use is preferably 1 to 20 mol, and more preferably 5 to 10 mol, per mol of the compound represented by formula (A).

From the standpoint of yield etc., the amount of phospholipase D for use is preferably 100 to 1500 U, and more preferably 300 to 700 U, per mol of the compound represented by formula (A). One U is defined as an enzyme amount with which 1 micro-mol of a substrate is changed per minute (µmol) under optimum conditions (an acidity at which the chemical reaction proceeds most at a temperature of 30°C) (1 micro-mol per minute).

This reaction is performed in the presence of a solvent. The solvent is not particularly limited, as long as the solvent can help phospholipase D exert its activity. The solvent preferable for use includes various buffers. A preferable buffer is an acetate buffer. The solvent preferably has a pH of 4 to 7, and more preferably 5 to 6. This reaction system may contain various organic solvents for dissolving the compound represented by formula (A) (e.g., ethyl acetate), in addition to the aqueous solvent.

This reaction is typically performed by mixing a solution of the compound represented by formula (A) in an organic solvent with a solution of the compound represented by formula (B) in an aqueous solvent, and adding phospholipase D to the mixture.

In this reaction, additives, in addition to the components described above, may also be suitably used to the degree that the progress of the reaction is not significantly interfered with.

The reaction temperature is not particularly limited, as long as the temperature allows phospholipase D to exert its activity. The reaction temperature is typically 20 to 50°C, and preferably 35 to 45°C.

The reaction time is not particularly limited, as long as the reaction time allows phospholipase D to exert its activity. The reaction time is typically 2 hours to 150 hours, preferably 8 hours to 100 hours, and more preferably 12 hours to 24 hours.

After completion of the reaction, the solvents are distilled off; and the product can be isolated and purified by typical techniques, such as chromatography and recrystallization. The structure of the product can be identified, for example, by element analysis, MS (FD-MS) analysis, IR analysis, ¹H-NMR, or ¹³C-NMR.

To improve lipid nanoparticle safety, ionizable lipids have been developed and nanoparticulated. Ionizable lipids are positively charged in the acidic range, and the change in net electrical charge in that case is 0 → +1. However, the change in net electrical charge of the phospholipid of the present invention (charge-reversible lipid) can be within the range of -1 to +2; the viewpoint is different. The phospholipid of the present invention is even ionized under neutral conditions, and differs from ionizable lipids in physicochemical properties. Because the lipid of the present invention is able to behave as an amphipathic lipid under neutral conditions, the lipid can offer the prospect of higher stability and higher safety.

The use of the phospholipid of the present invention enables the formation of a lipid particle that is not positively charged at a pH of the body fluid (typically in the neutral range), and that enables more efficient onset of the effect of a medicinal substance encapsulated in the lipid particle.

The phospholipid of the present invention can achieve the effects of the present invention even when it is in the form of a phospholipid salt. The phospholipid salt, which is a salt of the phospholipid of the present invention, is also one aspect of the present invention. In the present specification, the matter described as the phospholipid also includes the phospholipid salt.

### 2. Lipid Particle

The present invention, according to one embodiment thereof, relates to a lipid particle (which may be referred to as "the lipid particle of the present invention" in the present specification) containing the phospholipid of the present invention (which may be referred to as "phospholipid A" in the present specification). The following describes this lipid particle.

The lipid particle of the present invention is not particularly limited, as long as it contains the phospholipid of the present invention as a component lipid of the particle. The phospholipid of the present invention contained in the lipid particle may be one type alone, or a combination of two or more types. The lipid particle of the present invention is, for example, formed such that an amphipathic lipid containing the phospholipid of the present invention forms the outer layer, and the lipid molecules are lined with their hydrophilic portions facing outward. Examples of the lipid particle include particles having the outer layer formed from a lipid monolayer membrane, and particles having the outer layer formed from a lipid bilayer membrane. The lipid particle is preferably a particle having the outer layer formed from a lipid monolayer membrane, and more preferably a particle having the outer layer formed from a lipid monolayer membrane in which amphipathic lipid molecules are lined with their hydrophilic portions facing outward. The inner layer of the particle may be composed of a homogeneous aqueous phase or a homogeneous oil phase, and the inner layer preferably contains one or multiple reverse micelles.

The particle size of the lipid particle of the present invention is not particularly limited. The particle size is preferably nanosize; and is specifically, for example, 10 to 700 nm, preferably 20 to 500 nm, more preferably 30 to 250 nm, even more preferably 30 to 150 nm, still even more preferably 40 to 120 nm, and particularly preferably 50 to 100 nm.

The lipid particle of the present invention is not positively charged at a pH of the body fluid (typically in the neutral range). More specifically, the lipid particle of the present invention has a zeta potential of -80 to -1 mV, -50 to -1 mV, -40 to -1 mV, or -30 to -1 mV in a Tris-HCl buffer with a pH of 7.4.

The lipid particle of the present invention may contain other lipids as a lipid component of the particle, in addition to the phospholipid of the present invention. Specific examples of such lipids include phospholipids, glycolipids, sterols, saturated or unsaturated fatty acids, and the like.

Specific examples of phospholipids include phosphatidylcholines, such as dilauroylphosphatidylcholine, dimyristoylphosphatidylcholine, dipalmitoylphosphatidylcholine, distearoylphosphatidylcholine, dioleoylphosphatidylcholine, dilinoleoylphosphatidylcholine, myristoylpalmitoylphosphatidylcholine, myristoylstearoylphosphatidylcholine, and palmitoylstearoylphosphatidylcholine; phosphatidylglycerols, such as dilauroylphosphatidylglycerol, dimyristoylphosphatidylglycerol, dipalmitoylphosphatidylglycerol, distearoylphosphatidylglycerol, dioleoylphosphatidylglycerol, dilinoleoylphosphatidylglycerol, myristoylpalmitoylphosphatidylglycerol, myristoylstearoylphosphatidylglycerol, and palmitoylstearoylphosphatidylglycerol; phosphatidylethanolamines, such as dilauroylphosphatidylethanolamine, dimyristoylphosphatidylethanolamine, dipalmitoylphosphatidylethanolamine, distearoylphosphatidylethanolamine, dioleoylphosphatidylethanolamine, dilinoleoylphosphatidylethanolamine, myristoylpalmitoylphosphatidylethanolamine, myristoylstearoylphosphatidylethanolamine, and palmitoylstearoylphosphatidylethanolamine; phosphatidylserine; phosphatidic acid; phosphatidylinositol; sphingomyelin; cardiolipin; egg yolk lecithin; soybean lecithin; and hydrogenated products thereof. These phospholipids may be those modified with a water-soluble polymer, such as polyethylene glycol (which may be referred to below as "PEG"), and it is preferable that the phospholipids are not modified with a PEG lipid (i.e., do not contain a PEG lipid) from the standpoint that, in particular, in the case in which the water-soluble polymer is PEG, protein expression can be exhibited even without modification with PEG.

Specific examples of glycolipids include glyceroglycolipids, such as diglycosyl diglyceride, digalactosyl diglyceride, galactosyl diglyceride, and glycosyl diglyceride; glycosphingolipids, such as galactosyl cerebroside, and ganglioside; stearyl glucoside, esterified stearyl glycoside, and the like.

Specific examples of sterols include cholesterol, cholesteryl hemisuccinate, lanosterol, dihydrolanosterol, desmosterol, dihydrocholesterol, phytosterol, stigmasterol, timosterol, ergosterol, sitosterol, campesterol, brassicasterol, and the like. The lipid particle preferably contains a sterol as a lipid component of the liposome membrane, particularly because of its action to stabilize the liposome membrane, and to adjust the fluidity of the liposome membrane.

Specific examples of saturated or unsaturated fatty acids include saturated or unsaturated fatty acids having 10 to 22 carbon atoms, such as decanoic acid, myristic acid, palmitic acid, stearic acid, arachidonic acid, oleic acid, linoleic acid, linolenic acid, eicosapentaenoic acid, docosanoic acid, and docosahexaenoic acid.

These lipids may be used singly, or in a combination of two or more.

The lipid particle of the present invention preferably contains a phospholipid other than the phospholipid of the present invention (which may be referred to as "phospholipid B" in the present specification), and a sterol. Phospholipid B preferably has a saturated chain hydrocarbon group. Phospholipid B is, for example, preferably phosphatidylcholine, and particularly preferably dipalmitoylphosphatidylcholine. The sterol is, for example, preferably cholesterol.

When the lipid particle of the present invention contains phospholipid B, phospholipid B is present in an amount of, for example, 15 to 100 mol, preferably 30 to 70 mol, more preferably 40 to 60 mol, and even more preferably 45 to 55 mol, per 100 mol of the phospholipid of the present invention. Alternatively, phospholipid B is present in an amount of, for example, 5 to 70 mol, preferably 5 to 40 mol, more preferably 5 to 30 mol, and even more preferably 10 to 15 mol, per 100 mol of the phospholipid of the present invention.

When the lipid particle of the present invention contains a sterol, the sterol is present in an amount of, for example, 30 to 200 mol, preferably 60 to 140 mol, more preferably 80 to 120 mol, even more preferably 90 to 110 mol, and still even more preferably 95 to 105 mol, per 100 mol of the phospholipid of the present invention.

When the lipid particle of the present invention contains phospholipid B and a sterol, phospholipid B is present in an amount of, for example, 10 to 100 mol, preferably 10 to 70 mol, more preferably 15 to 40 mol, and even more preferably 20 to 30 mol, per 100 mol of the sterol. Alternatively, phospholipid B is present in an amount of, for example, 10 to 100 mol, preferably 10 to 70 mol, more preferably 15 to 40 mol, and even more preferably 20 to 30 mol, per 100 mol of the phospholipid of the present invention.

The phospholipid of the present invention and optionally added other lipids (in a preferable embodiment, phospholipid B and a sterol) are present in a total amount of, for example, 40 to 160 mol, preferably 50 to 150 mol, more preferably 60 to 140 mol, even more preferably 70 to 130 mol, and still even more preferably 80 to 125 mol, per 100 mol of the lipid component of the lipid particle of the present invention.

In the lipid particle of the present invention, part of the phospholipid may be modified with a water-soluble polymer, such as PEG. A phospholipid modified with PEG is present in an amount of, for example, 0 to 50 mol, preferably 0 to 30 mol, more preferably 0 to 20 mol, and even more preferably 0 to 15 mol, per 100 mol of the lipid component of the lipid particle of the present invention. However, from the standpoint that protein expression can be exhibited without modification with PEG, the lipid particle of the present invention does not need to be modified with a PEG lipid, i.e., the lipid particle of the present invention does not need to contain a PEG lipid.

In the lipid particle of the present invention, a medicinal substance is preferably encapsulated. The medicinal substance is not particularly limited, and examples include polynucleotides, peptides, proteins, carbohydrates, low-molecular compounds, and the like. The medicinal substance is preferably negatively charged, and preferably water-soluble. Such a medicinal substance suitably usable is a polynucleotide. The target disease for the medicinal substance is not particularly limited, and examples include infectious diseases (in particular, it can be suitably used as a vaccine against infectious diseases), cancer (in particular, solid cancer), and the like.

The polynucleotide is not particularly limited, as long as the polynucleotide can function as a medicinal substance. Examples include mRNA, siRNA, miRNA, antisense nucleic acids, expression vectors therefor, expression vectors for proteins, nucleic acids for genome editing (e.g., guide RNAs, Cas protein expression vectors, and TALEN expression vectors), nucleic acid vaccines, and the like.

The case in which the polynucleotide is mRNA is described below. In the present specification, the term "messenger RNA (mRNA)" refers to a polynucleotide encoding at least one polypeptide. mRNA as used herein encompasses both modified and unmodified RNA. mRNA may contain one or more coding and non-coding regions. mRNA can be purified from natural sources, produced using recombinant expression systems and optionally purified, chemically synthesized, etc. Where appropriate, for example, in the case of chemically synthesized molecules, mRNA can comprise nucleoside analogs, such as analogs having chemically modified bases or sugars, main chain modifications, etc. An mRNA sequence is presented in the 5' to 3' direction unless otherwise indicated. In some embodiments, mRNA is or comprises natural nucleosides (e.g., adenosine, guanosine, cytidine, uridine), nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine), chemically modified bases, biologically modified bases (e.g., methylated bases), intercalated bases, modified sugars (e.g., 2'-fluororibose, ribose, 2'-deoxyribose, arabinose, and hexose), and/or modified phosphate groups (e.g., phosphorothioates and 5'-N-phosphoramidite linkages).

The mRNA used in the present invention may comprise one or more nonstandard nucleotide residues. The nonstandard nucleotide residues may include, for example, 5-methyl-cytidine ("5mC"), pseudouridine ("ΨU"), and/or 2-thio-uridine ("2sU"). Incorporation of such residues into mRNA can be performed by a known method. The mRNA may be RNA that is defined as RNA in which 25% of U residues are 2-thio-uridine, and 25% of C residues are 5-methylcytidine. The above RNA can be used by a known method. The presence of nonstandard nucleotide residues may render an mRNA more stable and/or less immunogenic than a control mRNA with the same sequence but containing only standard residues. In further embodiments, the mRNA may comprise one or more nonstandard nucleotide residues chosen from isocytosine, pseudoisocytosine, 5-bromouracil, 5-propynyluracil, 6-aminopurine, 2-aminopurine, inosine, diaminopurine, and 2-chloro-6-aminopurine cytosine, as well as combinations of these modifications and other nucleobase modifications. Certain embodiments may further include additional modifications to the furanose ring or nucleobase. Additional modifications may include, for example, sugar modifications or substitutions (e.g., one or more of a 2'-O-alkyl modification, a locked nucleic acid (LNA)). In some embodiments, the RNAs may be complexed or hybridized with additional polynucleotides and/or peptide polynucleotides (PNA). In embodiments in which the sugar modification is a 2'-O-alkyl modification, such modification may include, but are not limited to, a 2'-deoxy-2'-fluoro modification, a 2'-O-methyl modification, a 2'-O-methoxyethyl modification, and a 2'-deoxy modification. In certain embodiments, any of these modifications may be present in 0 to 100% of the nucleotides, for example, 0%, 1%, 10%, 25%, 50%, 75%, 85%, 90%, or more than 95%, or 100% of the constituent nucleotides individually or in combination.

The medicinal substance is preferably contained in the inner layer of the lipid particle of the present invention. When the medicinal substance is a polynucleotide, the medicinal substance is preferably contained within a reverse micelle in the inner layer.

The weight ratio of the lipid component of the lipid particle of the present invention to the medicinal substance (the lipid component of the lipid particle of the present invention/the medicinal substance, wt/wt) is, for example, 0 to 500, preferably 0 to 300, more preferably 0 to 100, even more preferably 5 to 50, still even more preferably 10 to 40, and particularly preferably 15 to 35, when, for example, the medicinal substance is a polynucleotide, such as mRNA.

The lipid particle of the present invention may contain other components in addition to the components described above. Examples of other components include membrane stabilizers, charged substances, antioxidants, membrane proteins, polyethylene glycol (PEG), antibodies, peptides, sugar chains, and the like.

An antioxidant can be added to prevent oxidation of the membrane, and is optionally used as a component of a membrane. Examples of antioxidants used as a component of a membrane include butylated hydroxytoluene, propyl gallate, tocopherol, tocopherol acetate, mixed tocopherol concentrate, vitamin E, ascorbic acid, L-ascorbyl stearate, ascorbyl palmitate, sodium hydrogen sulfite, sodium sulfite, sodium edetate, erythorbic acid, citric acid, and the like.

A membrane protein can be added to add functions to a membrane, or to stabilize the structure of a membrane; and is optionally used as a component of a membrane. Examples of membrane proteins include peripheral membrane proteins, integral membrane proteins, albumin, and recombinant albumins.

The other components are present in an amount of, for example, 20% or less, and preferably 10% or less, based on a lipid particle solution containing the lipid particle of the present invention taken as 100 mass%.

The lipid particle of the present invention can be produced in accordance with or with reference to a known production method for a lipid particle. The lipid particle of the present invention can be produced preferably by a method including the step of mixing an alcohol solution containing the phospholipid of the present invention with an acidic aqueous solution containing a water-soluble medicinal substance (step 1).

The alcohol as a solvent of the alcohol solution is not particularly limited, as long as the alcohol can dissolve the phospholipid. From the standpoint of solubility, the alcohol is preferably ethanol or butanol, more preferably ethanol or t-butanol, and even more preferably ethanol.

The acidic aqueous solution typically contains an acid in addition to a water-soluble medicinal substance and water, which is a solvent. Examples of the acid include organic acids and inorganic acids, with organic acids being preferable. Examples of organic acids include maleic acid, formic acid, acetic acid, propionic acid, folic acid, isobutyric acid, valeric acid, isovaleric acid, pyruvic acid, oxalic acid, malonic acid, succinic acid, glutaric acid, ketoglutaric acid, adipic acid, lactic acid, tartaric acid, fumaric acid, oxaloacetic acid, malic acid, isocitric acid, citric acid, benzoic acid, phthalic acid, isophthalic acid, terephthalic acid, hemimellitic acid, trimellitic acid, trimesic acid, mellophanic acid, prehnitic acid, pyromellitic acid, mellitic acid, methanesulfonic acid, benzenesulfonic acid, p-toluenesulfonic acid, camphorsulfonic acid, p-toluenesulfinic acid, benzenesulfinic acid, and the like; with citric acid being preferable. Examples of inorganic acids include hydrochloric acid, sulfuric acid, nitric acid, carbonic acid, boric acid, boronic acid, hydrofluoric acid, hypochlorous acid, chlorous acid, chloric acid, perchloric acid, hypobromous acid, bromous acid, bromic acid, perbromic acid, hypoiodous acid, iodous acid, iodic acid, periodic acid, phosphorous acid, phosphoric acid, polyphosphoric acid, chromic acid, permanganic acid, and Amberlyst. The acids may be used singly, or in a combination of two or more.

The acidic aqueous solution preferably has a pH of 3 to 5.

The mixture ratio of the acidic aqueous solution to the alcohol solution (the acidic aqueous solution/the alcohol solution, v/v) is, for example, 1.5 to 10, preferably 2 to 8, more preferably 3 to 7, even more preferably 3.5 to 5.5, and still even more preferably 4 to 5.

Mixing is not particularly limited, as long as the mixing mode allows the phospholipid of the present invention to be mixed with the medicinal substance. Typically, mixing is performed using a reaction system with a microchannel. In this case, the conditions can be suitably adjusted according to the reaction system.

### 3. Application of Lipid Particle

The present invention, according to one embodiment thereof, relates to a medical drug containing the lipid particle of the present invention in which a medicinal substance is encapsulated (which may be referred to as "the medical drug of the present invention" in the present specification). The lipid particle of the present invention in which a medicinal substance is encapsulated is also usable as a reagent.

The lipid particle of the present invention enables a medicinal substance (e.g., a polynucleotide, such as mRNA) to exert its effect more efficiently, while reducing cytotoxicity. Thus, the lipid particle of the present invention can suitably be used as a carrier for a medicinal substance.

The content of the active ingredient (i.e., a medicinal substance) in the medical drug of the present invention can be suitably determined, taking into consideration, for example, the type of target disease, target therapeutic effect, administration method, treatment period, patient's age, and patient's body weight. For example, the content of the active ingredient in the medical drug of the present invention may be about 0.0001 parts by weight to 100 parts by weight, based on the entire medical drug of the present invention taken as 100 parts by weight.

The mode of administration of the medical drug of the present invention is not particularly limited, as long as a desired effect is brought about. The medical drug can be administered to mammals including humans through an administration route of either peroral administration or parenteral administration (e.g., intravenous injection, intramuscular injection, subcutaneous administration, rectal administration, transdermal administration, and local administration). The mode of administration is preferably parenteral administration. The dosage forms for peroral administration and parenteral administration and the production methods therefor are well known to those skilled in the art. Such dosage forms can be produced by mixing an active ingredient with a pharmaceutically acceptable carrier and other components, in accordance with a standard method.

The dosage form for parenteral administration includes injectable preparations (e.g., drip injectable drugs, intravenous injectable drugs, intramuscularly injectable drugs, subcutaneously injectable drugs, and intradermally injectable drugs), drugs for external use (e.g., ointments, cataplasms, and lotions), suppositories inhalants, eye drops, ophthalmic ointments, nasal drops, ear drops, liposome drugs, and the like. For example, an injectable preparation can be prepared by dissolving the lipid particle of the present invention in injectable distilled water; and a solubilizing agent, a buffer, a pH adjuster, a tonicity agent, a soothing agent, a preservative, a stabilizer, etc., can be optionally added thereto. The medical drug may be a freeze-dried formulation that is prepared into a drug when needed.

The medical drug of the present invention may further contain other medicinal agents effective in the treatment or prevention of diseases. The medical drug of the present invention may also optionally contain components, such as antiseptic drugs, antiphlogistics, cell activators, vitamins, and amino acids.

For the carrier for use in preparing the medical drug of the present invention, those typically used in this technical field, such as excipients, binders, disintegrants, lubricants, colorants, and flavoring agents, can be used; and stabilizers, emulsifiers, absorption promoters, surfactants, pH adjusters, antiseptics, antioxidants, fillers, moisturizers, surface activators, dispersants, buffers, preservatives, solubilizing agents, soothing agents, and the like can also optionally be used.

The dosage of the medical drug of the present invention can be determined by a practical physician, taking into consideration various factors, such as the administration route; type of disease; degree of symptoms; patient's age, gender, and body weight; severity of disease; pharmacological findings such as pharmacokinetics and toxicological characteristics; whether a drug delivery system is used; and whether the medical drug is administered as part of a combination with other medicinal substances. The dosage of the medical drug of the present invention may be, for example, about 1 µg/kg (body weight) to 10 g/kg (body weight) per day. The dose schedule of the medical drug of the present invention can also be determined while taking into consideration the same factors as those for the dosage. For example, the medical drug of the present invention can be administered in the dosage per day described above once daily to once per month.

### Examples

The following describes the present invention in detail with reference to Examples. However, the present invention is not limited to these Examples.

### Example 1

### Synthesis of 2-[2-[(Dimethylamino)ethyl]methylamino]ethyl 1,2-dioleoyl-sn-glycero-3-phosphate (Compound a)

Compound a represented by the following chemical formula was synthesized by the following synthesis method.

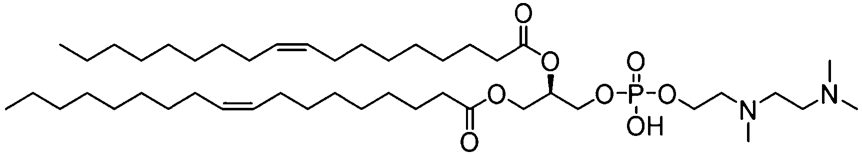

2-[[2-(Dimethylamino)ethyl]methylamino]ethanol (5.0 g) and sodium chloride (6.2 g) were dissolved in an acetate buffer (pH 5.5, 31.6 g), and then acetic acid (4.8 g) was added with stirring to prepare an amine-containing acetate buffer. Dioleoylphosphatidylcholine (1.5 g) was dissolved in ethyl acetate (18.2 g) at 40°C. Subsequently, the amine-containing acetate buffer (7.9 g) containing dissolved phospholipase D (2.8 mg, produced by Asahi Kasei Pharma Corporation) and ethyl acetate (12.0 g) were added. The mixture was stirred at 40°C for 19 hours. To the resulting solution, ethyl acetate, toluene, methanol and an aqueous sodium chloride solution were added, followed by stirring and standing. The organic layer was separated and then washed with an aqueous sodium chloride solution and methanol. The solvent was distilled off from the resulting organic layer under reduced pressure to obtain a crude product. The crude product was dissolved in ethyl acetate (7.5 mL) with heating. After cooling to room temperature, 4 M hydrogen chloride/ethyl acetate (1.4 mL) was added. Subsequently, acetone (35.0 mL) was added dropwise, and the mixture was stirred in an ice bath for 25 minutes. The resulting suspension was filtered, and the solid was rinsed with acetone and then vacuum-dried to obtain a solid. THF (13.0 mL) was added to the obtained solid, followed by stirring. Further, acetone (33.0 mL) was added dropwise, followed by cooling in an ice bath. The resulting suspension was filtered, and the solid was rinsed with acetone and vacuum-dried to obtain compound a (0.35 g).
Mass spectrometry (ESI): [M+H]⁺=830
¹H NMR (300 MHz, solvent CDCl₃) :δ=5.38-5.24 (m, 5H), 4.52-4.39 (m, 2H), 4.38-4.35 (m, 1H), 4.18-3.88 (m, 7H), 3.70-3.60 (m, 2H), 3.10 (s, 3H), 3.02 (s, 6H), 2.36-2.27 (m, 4H), 2.02-1.98 (m, 8H), 1.64-1.54 (m, 4H), 1.40-0.91 (m, 40H), 0.88 (t, J = 6.0 Hz, 6H).

The properties of the obtained phospholipid were evaluated as described below.

### Example 2

### Production of Lipid Particle Solution

Compound a synthesized in Example 1, dipalmitoylphosphatidylcholine, and cholesterol were added to ethanol in a molar ratio of 45:10:45, thereby preparing a phospholipid alcohol solution (lipid concentration: 0.99 mM). Gaussia luciferase mRNA or firefly luciferase mRNA was added to a 1 mM sodium citrate/citric acid aqueous solution (pH 4.5), thereby preparing an mRNA acidic aqueous solution. These two solutions were rapidly mixed at 25°C using a static micromixer (produced by YMC Co., Ltd.) so that the lipid solution and the mRNA solution were mixed in a volume ratio of 1:4.25 (mRNA:lipid = 1:30 wt/wt). Dialysis was performed with ultrapure water at 4°C for 8 hours to produce a lipid particle solution containing lipid particles.

### Comparative Example 1

### Production of Lipid Particle Solution

A lipid particle solution was produced in the same manner as in Example 2, except that dioleoylglycerophosphate-diethylenediamine (DOP-DEDA) was used in place of compound a.

The properties of the lipid particle solutions of Example 2 and Comparative Example 1 were evaluated under the following measurement conditions.

### Measurement of Average Particle Size of Lipid Particles

The lipid particle solutions obtained in Example 2 and Comparative Example 1 were diluted with RNase-free water, and the average particle size of the lipid particles was measured using a Litesizer 500 (produced by Anton Paar).

### Measurement of ζ-potential

The lipid particle solutions were diluted with a 10 mM Tris-HCl buffer (pH 7.4) or a 1 mM sodium citrate/citric acid aqueous solution (pH 4.5), and then the ζ-potential was measured using a Litesizer 500.

Table 1 shows the results.

**Table 1**

| Lipid particles solution | Luciferase mRNA | Average particle size (nm) | ζ-potential (mV) pH 7.4 | ζ-potential (mV) pH 4.5 | mRNA encapsulation efficiency (%) |
|---|---|---|---|---|---|
| Example 2 | Firefly | 69.0 | -8.6 | +19.5 | 87.3 |
| Example 2 | Gaussia | 74.0 | -25.3 | +17.1 | 90.3 |
| Comparative Example 1 | Gaussia | 155 | +2.0 | +20.4 | 76.6 |

### Example 3

### Production of Lipid Particle Solution

A lipid particle solution was prepared using firefly luciferase mRNA in the same manner as in Example 2. Further, the prepared lipid particle solution was ultrafiltered with Amicon Ultra-4 (produced by Millipore) to concentrate the lipid particle solution (final mRNA concentration: 25 ng/µL). After concentration, sucrose was added to the lipid particle solution so that the final sucrose concentration was 0.3 M, thereby producing the lipid particle solution of Example 3.

### Example 4

### Production of Lipid Particle Solution

Compound a, dipalmitoylphosphatidylcholine, cholesterol, and dimyristoyl glycerol-polyethylene glycol 2000 were added to ethanol in a molar ratio of 45:10:45:1.5, thereby preparing a lipid alcohol solution (lipid concentration: 0.99 mM). mRNA was added to a 1 mM sodium citrate/citric acid aqueous solution (pH 4.5), thereby preparing an mRNA acidic aqueous solution. These two solutions were rapidly mixed at 25°C using a static micromixer (produced by YMC Co., Ltd.) so that the lipid solution and the mRNA solution were mixed in a volume ratio of 1:4.25 (mRNA:lipid = 1:30 wt/wt). Dialysis was performed with ultrapure water at 4°C for 8 hours to produce a lipid particle solution.

### Comparative Example 2

### Production of Lipid Particle Solution

A lipid particle solution was prepared using firefly luciferase mRNA in the same manner as in Example 2, except that DOP-DEDA was used in place of compound a.

### Comparative Example 3

### Production of Lipid Particle Solution

A lipid particle solution was produced in the same manner as in Example 4, except that DOP-DEDA was used in place of compound a.

The properties of the lipid particle solutions of Examples 3 and 4 and Comparative Examples 2 and 3 were evaluated under the following measurement conditions.

### Luciferase Expression Test

50 µL of each lipid particle solution (corresponding to 1.25 µg of mRNA) was individually intramuscularly administered to the left thigh muscle of mice (BALB/c, female, 6 weeks old). The luciferase expression effect was evaluated using an IVIS Lumina System (produced by Xenogen) 4, 6, 8, and 24 hours after administration of each lipid particle solution. Ten minutes before imaging, D-luciferin potassium salt (produced by BioVision) was intraperitoneally administered in an amount of 150 mg/kg body weight, and the luminescence results were confirmed. Further, the administration site was used as the region of interest, and the total luminescence was quantified.

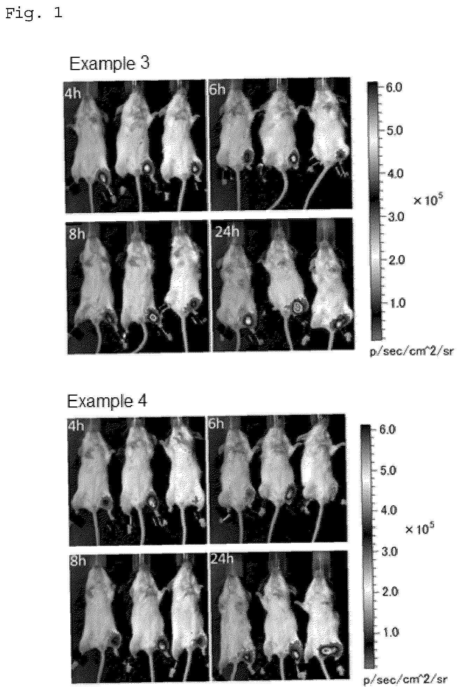

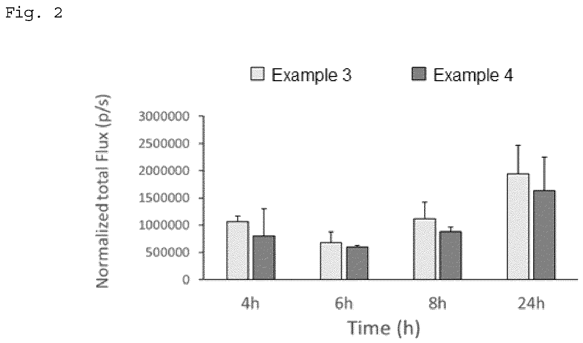

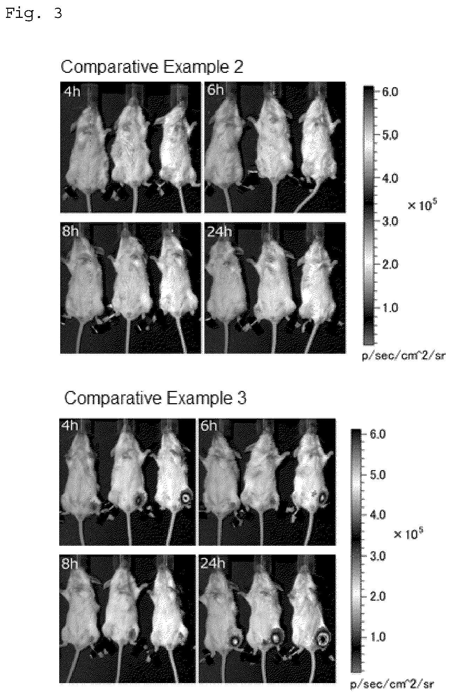

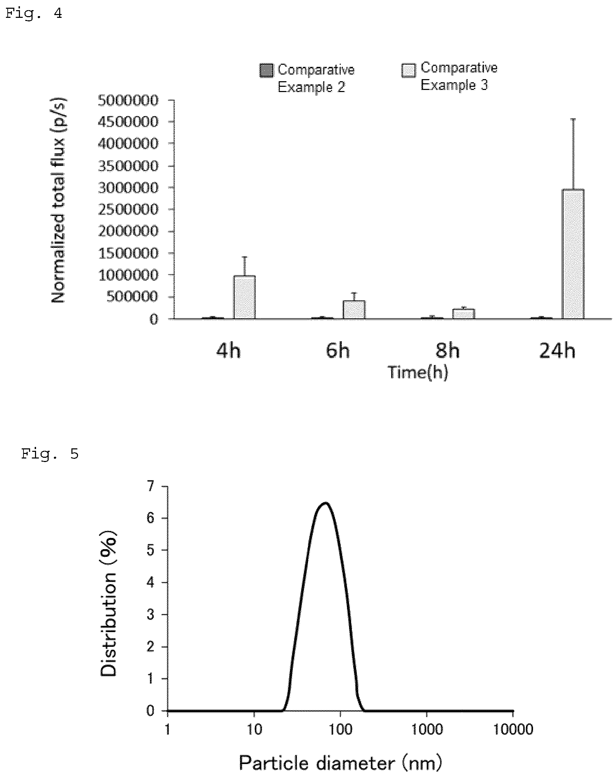

Fig. 1 shows the imaging results using Examples 3 and 4, and Fig. 2 is a graph of the total luminescence. Fig. 3 shows the imaging results using Comparative Examples 2 and 3, and Fig. 4 is a graph of the total luminescence.

The results shown in Figs. 1 and 2 show that luciferase expression was confirmed in Examples 3 and 4, both of which use compound a. That is, the results show that in a lipid particle solution containing the phospholipid of the present invention, luciferase expression does not depend on the presence or absence of a PEG lipid.

In contrast, the results shown in Figs. 3 and 4 show that luciferase expression was not observed in Comparative Example 2, in which DOP-DEDA is used and the lipid particle solution does not contain PEG. In Comparative Example 3, in which DOP-DEDA is used and the lipid particle solution contains PEG, luciferase expression was confirmed. The results show that in a lipid particle solution that does not contain the phospholipid of the present invention, luciferase expression depends on the presence or absence of a PEG lipid.

### Example 5

### Production of Lipid Particle Solution

A lipid particle solution was produced using firefly luciferase mRNA in the same manner as in Example 2.

### Comparative Example 4

### Production of Lipid Particle Solution

ALC-0315, distearoylphosphatidylcholine, and cholesterol were added to ethanol in a molar ratio of 46.3:9.4:42.7, thereby preparing a lipid alcohol solution (lipid concentration: 0.99 mM). HiBiT mRNA was added to a 1 mM sodium citrate/citric acid aqueous solution (pH 4.5), thereby preparing an mRNA acidic aqueous solution. These two solutions were rapidly mixed at 25°C using a static micromixer (produced by YMC Co., Ltd.) so that the lipid solution and the mRNA solution were mixed in a volume ratio of 1:3 (mRNA:lipid = 1:25.5 wt/wt). Dialysis was performed with ultrapure water at 4°C for 8 hours to produce a lipid particle solution containing lipid particles.

### Measurement of Particle Size Distribution of Lipid Particles

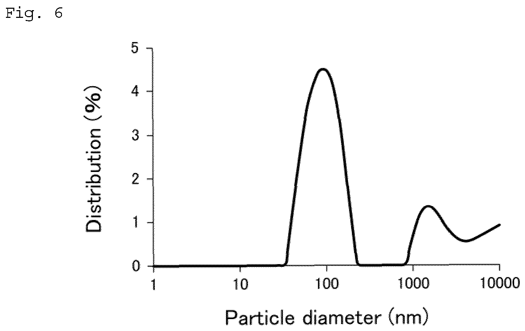

The lipid particle solutions produced in Example 5 and Comparative Example 4 were diluted with RNase-free water, and the particle size of the lipid particles was measured using a Litesizer 500 (produced by Anton Paar). Figs. 5 and 6 show the results.

The results shown in Figs. 5 and 6 show that lipid particles showing a sharp particle size distribution were formed in Example 5, which uses compound a. In Comparative Example 4, which uses ALC-0315, lipid particles with large particle sizes were also formed. That is, the results show that a lipid particle solution containing the phospholipid of the present invention makes it possible to produce lipid particles with a uniform particle size, even without a PEG lipid.

### Example 6

The toluene of [³H] cholesteryl hexadecyl ether dissolved in toluene was distilled off under reduced pressure, followed by dissolving in ethanol. The resulting solution was mixed with a phospholipid solution obtained by dissolving compound a, dipalmitoylphosphatidylcholine, cholesterol, and dimyristoyl glycerol-polyethylene glycol 2000 in ethanol in a molar ratio of 45:10:45:1.5, thereby preparing a lipid alcohol solution. OVA mRNA was added to a 50 mM acetate buffer (pH 5.0), thereby preparing an mRNA acidic aqueous solution. These two solutions were rapidly mixed at 25°C using a static micromixer (produced by YMC Co., Ltd.) so that the lipid solution and the mRNA solution were mixed in a volume ratio of 1:4.25 (mRNA:lipid = 1:30 wt/wt). Ultrafiltration at 4°C was performed using RNase-free water to remove the ethanol, thereby producing a lipid particle solution containing lipid particles.

### Example 7

A lipid particle solution was produced in the same manner as in Example 6, except that a phospholipid solution obtained by dissolving compound a, dipalmitoylphosphatidylcholine, and cholesterol in ethanol in a molar ratio of 45:10:45 was used in place of the phospholipid solution obtained by dissolving compound a, dipalmitoylphosphatidylcholine, cholesterol, and dimyristoyl glycerol-polyethylene glycol 2000 in ethanol in a molar ratio of 45:10:45:1.5.

### Comparative Example 5

A lipid particle solution was prepared in the same manner as in Example 6, except that DOP-DEDA was used in place of compound a.

### Comparative Example 6

SM-102, distearoylphosphatidylcholine, cholesterol, and dimyristoyl glycerol-polyethylene glycol 2000 were added to ethanol in a molar ratio of 50:10:38.5:1.5, thereby preparing a lipid alcohol solution (lipid concentration: 0.99 mM). OVA mRNA was added to a 6.25 mM acetate buffer (pH 5.0), thereby preparing an mRNA acidic aqueous solution. These two solutions were rapidly mixed at 25°C using a static micromixer (produced by YMC Co., Ltd.) so that the lipid solution and the mRNA solution were mixed in a volume ratio of 1:4.25 (mRNA:lipid = 1:19.35 wt/wt). Ultrafiltration at 4°C was performed using RNase-free water to remove the ethanol, thereby producing a lipid particle solution containing lipid particles.

### Evaluation of Biodistribution of Lipid Particles

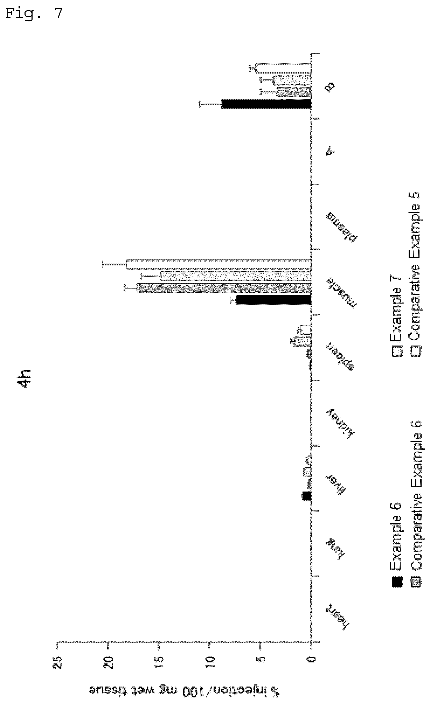

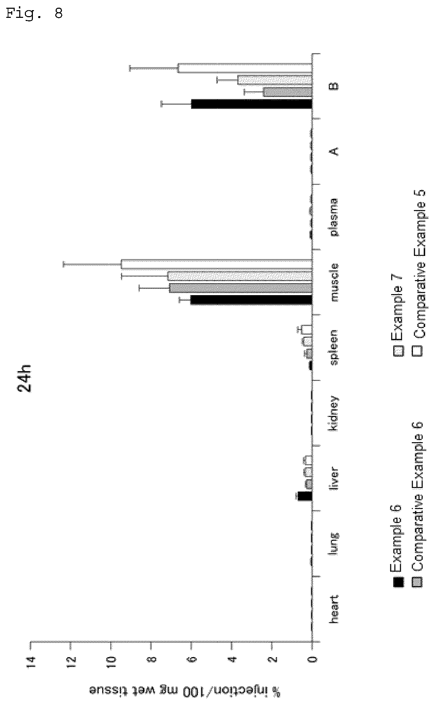

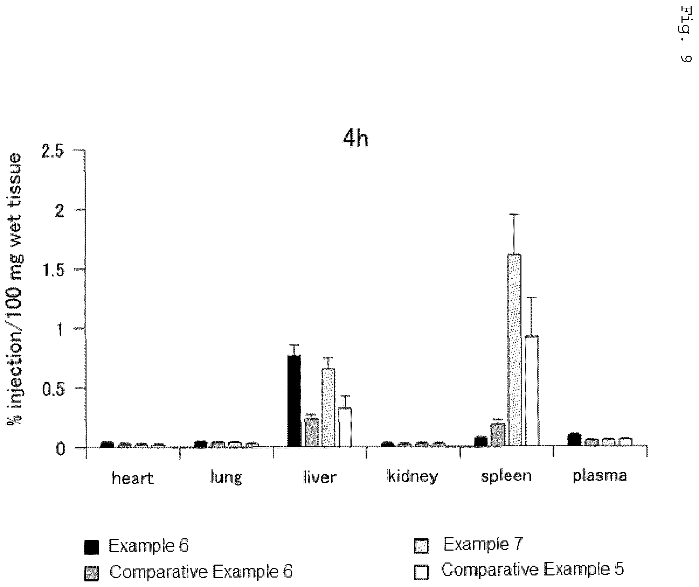

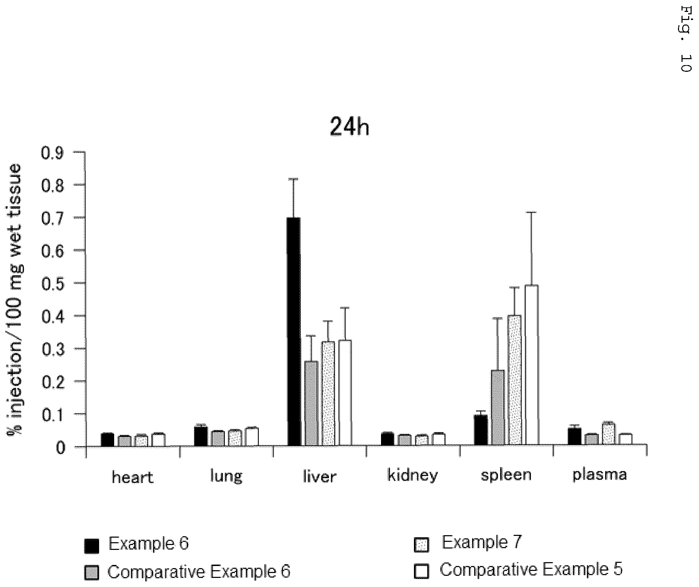

50 µL of each lipid particle solution was individually intramuscularly administered to the left thigh muscle of mice (C57BL/6J, female, 6 weeks old). 4 hours or 24 hours after administration of each lipid particle solution, the mice were exsanguinated under isoflurane anesthesia, and organs (heart, lung, liver, kidney, spleen, left thigh muscle, blood plasma, right subiliac lymph node (A), and left subiliac lymph node (B)) were removed and weighed. The liver was cut into pieces of about 100 mg. The blood was transferred to a tube and centrifuged at 3000 rpm for 10 minutes at 4°C. After centrifugation, 100 µL of blood serum was used as a measurement sample. Each organ and the blood serum were placed in vials, and 1 mL of a solvable solution was added, and the vials were allowed to stand in an incubator at 50°C overnight. 1 mL of a hydrogen peroxide solution as a decolorizing agent and 1 mL of 2-propanol as an antifoaming agent were added, and the vials were allowed to stand for several hours. 10 mL of Hionic Fluor was added, and each mixture was shaken and then allowed to stand in the dark for 1 hour. Subsequently, the radioactivity of each sample was measured using a liquid scintillation counter (LSC-8000, produced by Aloka). Figs. 7 and 8 show the results measured at 4 and 24 hours. For reference, Figs. 9 and 10 are diagrams in which the left thigh muscle, the right subiliac lymph node (A), and the left subiliac lymph node (B) are deleted from Figs. 7 and 8, and the values on the vertical axis are changed.

## Claims

1. A phospholipid represented by formula (1): 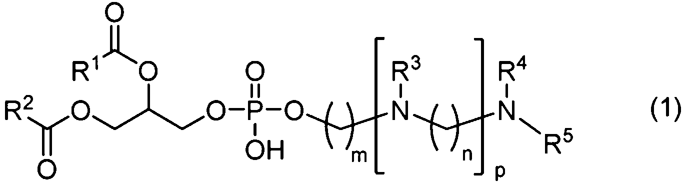 wherein R¹ and R² are the same or different, and each represents a chain hydrocarbon group; R³, R⁴, and R⁵ are the same or different, and each represents a C₁-C_{S} hydrocarbon group; m represents 1 or 2; n represents 1 or 2; and p represents a natural number of 1 to 3.

2. The phospholipid according to claim 1, wherein R³, R⁴, and R⁵ are the same or different, and each represents a C₁-C₃ hydrocarbon group.

3. The phospholipid according to claim 1 or 2, wherein m and n are 2.

4. The phospholipid according to claim 1 or 2, wherein p is 1.

5. The phospholipid according to claim 1 or 2, wherein R¹ and R² are the same or different, and each represents a C₁₂-C₂₄ chain hydrocarbon group.

6. The phospholipid according to claim 1 or 2, wherein the chain hydrocarbon group is an unsaturated chain hydrocarbon group.

7. A phospholipid salt that is a salt of the phospholipid of claim 1.

8. A lipid particle comprising the phospholipid of claim 1 (phospholipid A).

9. The lipid particle according to claim 8, in which a medicinal substance is encapsulated.

10. The lipid particle according to claim 9, wherein the medicinal substance is a polynucleotide.

11. The lipid particle according to claim 10, wherein the polynucleotide is messenger RNA.

12. The lipid particle according to claim 8, wherein the lipid particle comprises a sterol.

13. The lipid particle according to claim 8, wherein the lipid particle is free of a polyethylene glycol (PEG) lipid.

14. The lipid particle according to claim 8, wherein the lipid particle further comprises a phospholipid other than phospholipid A (phospholipid B).

15. An alcohol solution comprising the phospholipid of claim 1.

16. The alcohol solution according to claim 15, wherein an alcohol in the alcohol solution is ethanol.

17. A method for producing a lipid particle, the method comprising mixing the alcohol solution of claim 15 with an acidic aqueous solution containing a water-soluble medicinal substance.

18. A medical drug comprising the lipid particle of claim 8.
